(19) Europäisches Patentamt
European Patent Office
Office européen des brevets

(11) Veröffentlichungsnummer: **0 110 219**
**B1**

(12) # EUROPÄISCHE PATENTSCHRIFT

(45) Veröffentlichungstag der Patentschrift: **11.06.86**

(21) Anmeldenummer: **83111277.6**

(22) Anmeldetag: **11.11.83**

(51) Int. Cl.[4]: **C 07 D 275/04,** C 07 D 275/06, C 07 D 513/04, C 07 D 495/04, C 07 D 209/46, C 07 D 209/48, A 61 K 31/40, A 61 K 31/425

(54) **Heterocyclisch substituierte Nitrile, ihre Herstellung und Verwendung als Arzneimittel.**

(30) Priorität: **18.11.82 DE 3242477**

(43) Veröffentlichungstag der Anmeldung: **13.06.84 Patentblatt 84/24**

(45) Bekanntmachung des Hinweises auf die Patenterteilung: **11.06.86 Patentblatt 86/24**

(84) Benannte Vertragsstaaten: **AT BE CH DE FR GB IT LI NL SE**

(56) Entgegenhaltungen:
**CH - A - 621 340**
**DE - A - 3 141 198**
**DE - B - 1 135 461**
**US - A - 4 115 398**
**US - A - 4 325 963**

**BEILSTEINS HANDBUCH DER ORGANISCHEN CHEMIE, 4. Auflage, zweites Ergänzungswerk, 21. Band, 1953, SPRINGER-VERLAG, Berlin-Göttingen-Heidelberg, Seiten 358,359**
**Russian Chemical Reviews 30, 584 (1961)**

(73) Patentinhaber: **BASF Aktiengesellschaft, Carl-Bosch-Strasse 38, D-6700 Ludwigshafen (DE)**

(72) Erfinder: **Franke, Albrecht, Dr., Mandelring 11, D-6706 Wachenheim (DE)**
Erfinder: **Steiner, Gerd, Dr., Oberer Waldweg 1, D-6719 Kirchheim (DE)**
Erfinder: **Hofmann, Hans-Peter, Dr., Untere Hart 12, D-6703 Limburgerhof (DE)**
Erfinder: **Mueller, Claus-Dieter, Dr., Odenwaldring 84, D-6806 Viernheim (DE)**
Erfinder: **Teschendorf, Hans-Juergen, Dr., Georg-Nuss-Strasse 5, D-6724 Dudenhofen (DE)**

**EP 0 110 219 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER, STOCKHOLM 1986

**Beschreibung**

Die Erfindung betrifft heterocyclisch substituierte Nitrile, Verfahren zu deren Herstellung sowie diese enthaltende therapeutische Mittel und deren Verwendung bei der Bekämpfung von Krankheiten.

Es ist bekannt, daß der Wirkstoff Supidimid (3-(2,3-Dihydro-1,1-dioxido-3-oxo-1,2-benzisothiazol-2-yl)-2-oxo-piperidin; Arzneim. Forsch. *32,* 1101 (1982)) nützliche Eigenschaften als Tranquilizer und Hypnotikum aufweist.

Weiter sind in Beilsteins Handbuch der Organischen Chemie, 4. Auflage, Zweites Ergänzungswerk, Band 21 (1953), Seiten 358–359 sowie in Russian Chemical Reviews *30,* 584 (1961) Phthalimidoalkansäure nitrile ohne irgendeine Wirkungsangabe beschrieben worden.

Es wurde nun gefunden, daß heterocyclisch substituierte Nitrile der Formel I

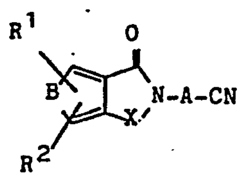 I,

in der
X $SO_2$, CO, S, SO oder $CH_2$,
A einen Alkenylenrest mit bis zu 6 C-Atomen,
B -CH=CH- oder S und
$R^1$ und $R^2$ Wasserstoff, Halogen, $C_{1-3}$-Alkyl, $C_{1-3}$-Alkoxy, Nitro und Trifluormethyl bedeuten, wertvolle pharmakologische Eigenschaften besitzen.

Die Gruppe X ist vorzugsweise $SO_2$ und CO, A bedeutet vorzugsweise 2-Methyl-buten-2-ylen und insbesondere 2-Methyl-propen-2-ylen (A-CN ist in diesen Fällen $CH_2$-$CH_2$-C($CH_3$)=CH-CN bzw. $CH_2$-C($CH_3$)=CH-CN), B ist vorzugsweise -CH=CH- und die Reste $R^1$ und $R^2$ bedeuten vorzugsweise Wasserstoff, Chlor und Fluor.

Es sei darauf hingewiesen, daß die erfindungsgemäßen Verbindungen als cis, trans-Isomere vorliegen können, wenn A ein Alkenylenrest ist.

Die folgenden Verbindungen sind als besonders wirksam zu nennen:
3-(2,3-Dihydro-1,1-dioxido-3-oxo-1,2-benzisothiazol-2-yl)-1-cyano-2-methyl-propen-1,
3-(2,3-Dihydro-1,1-dioxido-3-oxo-4-chlor-1,2-benzisothiazol-2-yl)-1-cyano-2-methyl-propen-1,
3-(2,3-Dihydro-1,1-dioxido-3-oxo-5-fluor-1,2-benzisothiazol-2-yl)-1-cyano-2-methyl-propen-1,
3-(2,3-Dihydro-1,1-dioxido-3-oxo-5-chlor-1,2-benzisothiazol-2-yl)-1-cyano-2-methyl-propen-1,
4-(2,3-Dihydro-1,1-dioxido-3-oxo-1,2-benzisothiazol-2-yl)-1-cyano-2-methyl-buten-1,
4-Phthalimido-2-yl-1-cyano-2-methyl-buten-1.

Die neuen Verbindungen lassen sich herstellen, indem man
a) eine Verbindung der Formel II

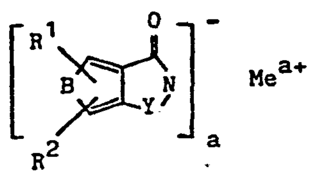 II,

worin Y dasselbe wie X ausgenommen SO ist, Me ein Alkali- oder Erdalkalimetall der Wertigkeit a ist und B, $R^1$ und $R^2$ die angegebene Bedeutung besitzen, mit einer Verbindung der Formel III

Hal-A-CN III,

worin A die angegebene Bedeutung hat und Hal ein Halogenatom darstellt, oder
b) — falls A einen gegebenenfalls methylverzweigten Alkenylenrest darstellt — eine Verbindung der Formel IV

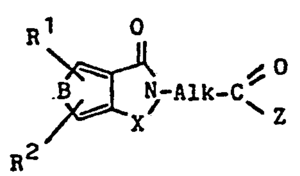 IV,

in der X, B, $R^1$ und $R^2$ die angegebenen Bedeutungen haben, Alk ein Alkylrest mit 1 bis 3 C-Atomen und Z Wasserstoff oder Methyl ist, mit einem Phosphonat der Formel V

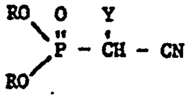 V,

in der R einen Alkylrest mit 1 bis 3 C-Atomen darstellt, und Y Wasserstoff oder Methyl bedeutet, umsetzt und in den so erhaltenen Verbindungen ein gegebenenfalls vorhandenes Schwefelatom — falls gewünscht — oxidiert.

Die für das Verfahren a) verwendeten Alkali- bzw. Erdalkalisalze erhält man aus den freien Basen mit Hilfe einer starken Alkali- bzw. Erdalkalimetall-Base, wie dem Hydroxid, Alkoholat, Hydrid oder einer entsprechenden metallorganischen Verbindung, vorzugsweise Natriumhydrid, in einem inerten organischen Lösungsmittel wie einem cyclischen gesättigten Ether, insbesondere Tetrahydrofuran oder Dioxan, oder einem polaren aprotischen Lösungsmittel, vorzugsweise Dimethylformamid.

Die Umsetzung der so erhaltenen Salze mit den Verbindungen der Formel III erfolgt dann zweckmäßig bei Temperaturen von 0 bis 150°C und ist im allgemeinen innerhalb von 3 bis 10 Stunden beendet. Als Lösungsmittel eignen sich die oben genannten inerten organischen Lösungsmittel, vorzugsweise Dimthylformamid.

Die Umsetzung b) wird unter den Bedingungen der Wittig-Horner-Reaktion in einem inerten Lösungsmittel in Gegenwart von einem Moläquivalent einer Base, bevorzugt Natriumalkoholat, Natriumhydrid oder Natriumamid, bei Temperaturen von 20 bis 80°C durchgeführt.

Als inerte Lösungsmittel eignen sich beispielsweise niedere Alkohole, wie Methanol, Ethanol oder Propanol, oder Ether, wie Diethylether, Tetrahydrofuran oder Dioxan oder polare aprotische Lösungsmittel wie Dimethylformamid. Letzeres ist bevorzugt.

Die Verbindungen der Formel I, in denen X ein Schwefelatom darstellt, können durch Oxidation in die entsprechenden SO- bzw. $SO_2$-Verbindungen übergeführt werden. Die Oxidation zur SO-Verbindung gelingt beispielsweise durch Zugabe von 3-Chlor-peroxybenzoesäure in einem inerten organischen Lösungsmittel, wie einem Halogenkohlenwasserstoff oder einem Ether.

Die Anlagerung von Wasserstoff an eine in der Seitenkette vorhandene Doppelbindung erfolgt am besten katalytisch mit Wasserstoff. Als Katalysatoren eignen sich Edelmetalle wie Palladium auf Kohlenstoff oder Platin. Die Reaktion kann bei Raumtemperatur und Normaldruck durchgeführt werden. Als Lösungsmittel kommen niedere Alkohole – wie Methanol oder Ethanol-, cyclische gesättigte Ether – wie Tetrahydrofuran oder Dioxan-, Ester – wie Essigester – oder dipolare aprotische Amide – wie Dimethylformamid – in Betracht.

Die Verbindungen der Formel I werden in der Mehrzahl der Fälle in Form von Kristallen erhalten und können durch Umkristallisation aus den üblichen organischen Lösungsmitteln, bevorzugt aus einem niederen Alkohol, wie Ethanol oder einem niedrigen Ester, vorzugsweise Essigsäureethylester, umkristallisiert oder durch Säulenchromatographie gereinigt werden.

Die erfindungsgemäßen Verbindungen weisen wertvolle pharmakologische Eigenschaften auf. Sie eignen sich beispielsweise zur Behandlung von psychischen Störungen, insbesondere von Depressionen, sowie als Sedativa und Tranquilizer.

Zur Analyse der pharmakologischen Eigenschaften wurden folgende Wirkqualitäten bestimmt:

Sedative Wirkung

Gruppen von jeweils drei weiblichen NMRI-Mäusen erhielten die Prüfsubstanzen oral appliziert. Die photoelektrische Bestimmung der durch eine neue Umgebung induzierten Orientierungshypermotilität erfolgt 30 min nach Substanzapplikation für eine Dauer von 30 min.

Als $ED_{50}$ wurde die Dosis bestimmt, welche im Vergleich zu unbehandelten Kontrolltieren eine Abnahme der Orientierungshypermotilität um 50% bewirkt.

Antidepressive Wirkung

Reserpin (2,15 mg/kg s.c.) verursacht an Mäusen (Stamm: Swiss; männlich, 20 bis 26 g Gewicht) eine Senkung der Körpertemperatur um durchschnittlich 3°C, gemessen 2 h nach Reserpingabe bei einer Umgebungstemperatur von 20 bis 22°C. Antidepressiva hemmen diese Hypothermie dosisabhängig. Die Prüfsubstanzen wurden oral 60 Minuten vor Reserpin appliziert.

Als $ED_{50}$ wurde aus der linearen Regression zwischen log Dosis (mg/kg) und relativer Abnahme der Hypothermie die Dosis ermittelt, welche die durch Reserpin induzierte Hypothermie um 50% hemmt.

Von Supidimid sind schwache zentrale dämpfende Wirkungen bekannt, die sich auch in unserem Test auf sedative Wirkung zeigen. Die neuen Substanzen erweisen sich in der sedativen Wirkung (Tabelle) bis zu 10fach stärker wirksam als Supidimid.

Darüber hinaus weisen die neuen Verbindungen überraschende antidepressive Wirkungen auf, während diese Wirkqualität dem Supidimid bis zur maximalen Prüfdosis von 2150 mg/kg p.o. völlig fehlt. Die neuen Substanzen sind dagegen bereits in niedrigen Dosen wirksam (Tabelle) und übertreffen in der Wirkstärke das Standard-Antidepressivum Imipramin deutlich.

Im Vergleich zur sedativen Wirkung ist die antidepressive Wirkung der neuen Substanzen in deutlich – bis zu ca. 90fach – niedrigerer Dosis nachweisbar. Der antidepressive Effekt der neuen Verbindungen steht eindeutig im Vordergrund des Wirkungsspektrums.

**Tabelle**

| Substanz des Beispiels Nr. | Antidepressive Wirkung (Maus) $ED_{50}$ (mg/kg p.o.) | Sedative Wirkung (Maus) $ED_{50}$ (mg/kg p.o.) |
|---|---|---|
| 1+2 | 3,5 | 50 |

| | | |
|---|---|---|
| 4 | 3,1 | 52 |
| 6 | 1,1 | 100 |
| 11 | 18,9 | 68 |
| 12 | 11,4 | 49 |
| 18 | 4,5 | 29 |
| Supidimid | >2150 | 277 |
| Imipramin | 6,8 | ca. 100 |

Gegenstand der vorliegenden Erfindung sind daher weiter Arzneimittel, die eine Verbindung der Formel I enthalten, sowie die Verwendung der neuen Verbindungen bei der Behandlung von Krankheiten.

Die neuen Verbindungen können in den üblichen galenischen Applikationsformen, fest oder flüssig, angewendet werden, wie Tabletten, Filmtabletten, Kapseln, Pulver, Granulate, Dragees, Suppositorien oder Lösungen. Diese werden in üblicher Weise hergestellt. Die Wirkstoffe können dabei mit den üblichen galenischen Hilfsmitteln wie Tablettenbindern, Füllstoffen, Konservierungsmitteln, Tablettensprengmitteln, Fließregulierungsmitteln, Weichmachern, Netzmitteln, Dispergiermitteln, Emulgatoren, Lösungsmitteln, Retardierungsmitteln und/oder Antioxidantien verarbeitet werden (vgl. H. Sucker et al: Pharmazeutische Technologie, Thieme-Verlag, Stuttgart, 1978). Die so erhaltenen Applikationsformen enthalten den Wirkstoff normalerweise in einer Menge von 0,1 bis 99 Gew.%.

Die Dosierung der erfindungsgemäßen Verbindungen hängt vom Alter, Zustand und Gewicht des Patienten sowie von der Applikationsart ab. In der Regel beträgt z.B. bei oraler Gabe die tägliche Wirkstoffdosis 5 bis 300 mg.

Die vorliegende Erfindung wird durch die nachfolgenden Ausführungsbeispiele näher erläutert.

**Beispiel 1**

a) Herstellung des Ausgangsmaterials

20,0 g (250 mM) Dimethylacrylinitril wurden in 200 ml Tetrachlorkohlenstoff gelöst und portionsweise mit 49,0 g (282 mM) N-Bromsuccinimid unter gutem Rühren versetzt.

Nach Zugabe einer Spatelspitze Azo-bis-isobutyronitril, erhitzte man das Reaktionsgemisch langsam auf 80°C. Nach 4 h ließ man abkühlen und saugte das ausgefallene Succinimid ab. Das Filtrat wurde zur Trockene eingeengt. Man isolierte 39,7 g (99%) 3-Brom-2-methyl-3-cyano-propen-1 als bräunliches Öl, das genügend rein für die weitere Umsetzung ist.

b) Herstellung des Endprodukts

In eine Lösung von 32,8 g (164 mM) Saccharin-Na-Salz in 300 ml Dimethylformamid wurden 41,3 g (258 mM) 3-Brom-2-methyl-3-cyano-propen-1 langsam unter gutem Rühren zugetropft (leicht exotherme Reaktion). Die Reaktionsmischung wurde anschließend 5 h bei 80°C gerührt. Nach dem Abkühlen schüttete man das Reaktionsgemisch auf 3 l Eiswasser. Der Niederschlag wurde abgesaugt und mit Wasser gut ausgewaschen. Man isolierte 40,3 g helle Kristalle, die aus 340 ml Ethanol unter Zusatz von Aktivkohle umkristallisiert wurden. Ausbeute: 30,2 g (70%) 3-(2,3-Dihydro-1,1-dioxido-3-oxo-1,2-benzisothiazol-2-yl)-1-cyano-2-methyl-propen-1, Schmp. 119 bis 121°C. Das $^1$H-NMR-Spektrum zeigt ein cis, trans-Isomerenverhältnis von 2:1.

**Beispiel 2**

a) Herstellung des Ausgangsmaterials

Zu 40,0 g (200 mM) Saccharin-Na-Salz tropfte man unter Rühren langsam (exotherme Reaktion) 26 ml (324 mM) Chloraceton. Man steigerte anschließend allmählich die Temperatur bis auf 110°C und rührte die Reaktionsmischung 5 h bei dieser Temperatur. Nach dem Abkühlen versetzte man mit einem Überschuß Eiswasser und ließ noch 1 h nachrühren. Die ausgefallenen Festkörper wurden abgesaugt und aus Ethanol umkristallisiert. Nach Trocknen im Vakuumtrockenschrank bei 50°C isolierte man 35,2 g (74%) N-Acetonylsaccharin, Schmp. 142 bis 143°C.

b) Herstellung des Endprodukts

Zu 10,0 g (42 mM) N-Acetonyl-saccharin in 200 ml Dimethylformamid tropfte man gleichzeitig 7,4 g (42 mM) Diethylcyanmethyl-phosphonat und 6,8 g (38 mM) 30%iges Natriummethylat unter gutem Rühren hinzu (Temperaturerhöhung auf 34°C). Die Reaktionsmischung ließ man 1 h bei Raumtemperatur nachrühren. Anschließend goß man den Kolbeninhalt auf Eiswasser, extrahierte dreimal mit Methylenchlorid, trocknete die organische Phase und engte im Vakuum ein. Das Rohprodukt wurde aus Ethanol umkristallisiert. Man isolierte 2,5 g (23%) 3-(2,3-Dihydro-1,1-dioxido-3-oxo-1,2-benzoisothiazol-2-yl)-1-cyano-2-methyl-propen-1, Schmp. 118 bis 121°C.

**Beispiel 3**

10,0 g (66 mM) 2,3-Dihydro-3-oxo-1,2-benzisothiazol wurden in 140 ml Dimethylformamid suspendiert und portionsweise mit 2,0 g (66 mM) Natriumhydrid (80%ig) unter gutem Rühren versetzt. Nach 15 min tropfte man 12,8 g (80 mM) 3-Brom-2-methyl-3-cyano-propen-1 langsam hinzu und ließ die Reaktionsmischung 2 h bei 80°C

rühren. Nach dem Abkühlen destillierte man das Lösungsmittel im Vakuum ab, nahm den Ansatz in Eiswasser auf, extrahierte mit Methylenchlorid, wusch mit $H_2O$ gründlich aus, trocknete die organische Phase und engte ein. Das Rohprodukt (16 g dunkles Öl) wurde anschließend durch Säulenchromatographie (Kieselgel, Laufmittel Methylenchlorid/Methanol 95/5) gereinigt. Man isolierte 3,5 g (23%) 3-(2,3-Dihydro-3-oxo-1,2-benzisothiazol-2-yl)-1-cyano-2-methyl-propen-1, Schmp. 128 bis 130°C.

Analog Beispiel 3 wurden hergestellt:

4. 3-(2,3-Dihydro-4-chlor-1,1-dioxido-3-oxo-1,2-benzisothiazol-2-yl)-1-cyano-2-methyl-propen-1, Schmp. 127 bis 128°C.
5. 3-(2,3-Dihydro-5-fluor-1,1-dioxido-3-oxo-1,2-benziosothiazol-2-yl)-1-cyano-2-methyl-propen-1, Schmp. 125 bis 127°C.
6. 3-(2,3-Dihydro-5-chlor-1,1-dioxido-3-oxo-1,2-benzisothiazol-2-yl)-1-cyano-2-methyl-propen-1, Schmp. 120 bis 122°C.
7. 3-(2,3-Dihydro-4-methoxy-1,1-dioxido-3-oxo-1,2-benzisothiazol-2-yl)-1-cyano-2-methyl-propen-1, Schmp. 108 bis 110°C.
8. 3-(2,3-Dihydro-4-methozy-7-nitro-1,1-dioxido-3-oxo-1,2-benzisothiazol-2-yl)-1-cyano-2-methyl-propen-1, Schmp. 72 bis 73°C.
9. 3-(2,3-Dihydro-4-chlor-7-nitro-1,1-dioxido-3-oxo-1,2-benzisothiazol-2-yl)-1-cyano-2-methyl-propen-1, Schmp. 80 bis 82°C.
10. 3-(2,3-Dihydro-4-chlor-5-brom-1,1-dioxido-3-oxo-1,2-benzisothiazol-2-yl)-1-cyano-2-methyl-propen-1, Schmp. 195 bis 196°C.
11. 3-(Phthalimid-2-yl)-1-cyano-2-methyl-propen-1, Schmp. 139 bis 140°C.
12. 3-(Phthalimidin-2-yl)-1-cyano-2-methyl-propen-1, Schmp. 142 bis 144°C.
13. 3-(2,3-Dihydro-1,1-dioxido-3-oxo-1,2-thieno[3,4-d]isothiazol-2-yl)-1-cyano-2-methyl-propen-1, Schmp. 153 bis 154°C.

**Beispiel 14**

Zu 20,0 g (100 mM) Saccharin-Na-Salz in 100 ml abs. Dimethylformamid tropfte man 18,2 g (150 mM) 2-Cyano-4-chlor-buten-2 bei 40°C unter gutem Rühren langsam zu. Man ließ das Reaktionsgemisch 4 h bei 40°C nachrühren. Anschließend filtrierte man das abgeschiedene Kochsalz ab und engte das Filtrat im Vakuum auf etwa 30 ml ein. Nach Zufügen von 400 ml Wasser wurde das zuerst anfallende Öl langsam kristallin. Die Kristalle wurden abgesaugt, aus Ethanol umkristallisiert und bei 50°C im Vakuumtrockenschrank getrocknet. Ausbeute 17,4 g (66%) 4-(2,3-Dihydro-1,1-dioxido-3-oxo-1,2-benzisothiazol-2-yl)-2-cyano-buten-2, Schmp. 107 bis 108°C.

Analog Beispiel 14 wurden hergestellt:

15. 4-(2,3-Dihydro-1,1-dioxido-3-oxo-1,2-thieno[3,4-d]-isothiazol-2-yl)-2-cyano-buten-2, Schmp. 154 bis 155°C.
16. 4-(Phthalimid-2-yl)-2-cyano-buten-2, Schmp. 101 bis 102°C.

**Beispiel 17**

a) Herstellung des Ausgangsmaterials

Zu 10,0 g (50 mM) Saccharin-Na-Salz in 100 ml abs. Dimethylformamid tropfte man unter Rühren langsam 9,0 g (76 mM) 4-Chlor-butanon-2. Man steigerte anschließend allmählich die Temperatur bis auf 80°C und rührte die Reaktionsmischung 5 h bei dieser Temperatur. Nach dem Abkühlen versetzte man mit einem Überschuß Eiswasser und ließ noch 1 h nachrühren. Die ausgefallenen Festkörper wurden abgesaugt und aus Ethanol umkristallisiert. Nach Trocknen im Vakuumtrockenschrank bei 50°C isolierte man 10,9 g (86%) N-(2-Oxo-but-4-yl)-saccharin, Schmp. 118 bis 119°C.

b) Herstellung des Endprodukts

Zu 10,0 g (40 mM) N-(2-Oxo-but-4-yl)-saccharin in 150 ml absolutem Dimethylformamid tropfte man gleichzeitig 9,5 g (54 mM) Diethyl-cyanmethylphosphonat und 8,7 g (48 mM) 30%iges Natriummethylat unter gutem Rühren hinzu. Die Reaktionsmischung ließ man 5 h bei 40°C nachrühren. Anschließend goß man den Kolbeninhalt auf Eiswasser und saugte die ausgefallenen Kristalle ab. Nach Umkristallisieren aus Ethanol isolierte man 2,2 g (20%) 4-(2,3-Dihydro-1,1-dioxido-3-oxo-1,2-benzisothiazol-2-yl)-1-cyano-2-methyl-buten-1, Schmp. 117 bis 118°C.

Analog wurde hergestellt:

18. 4-(Phthalimid-2-yl)-1-cyano-2-methyl-buten-1, Schmp. 130 bis 131°C.

**Beispiel 19**

5,8 g (26 mM) 3-(2,3-Dihydro-3-oxo-1,2-benzisothiazol-2-yl)-1-cyano-2-methyl-propen-1 (Beispiel 3) wurde in 160 ml Methylenchlorid gelöst und mit 5,4 g (26 mM) 3-Chlor-peroxybenzoesäure (85%ig) unter gutem Rühren versetzt. Man ließ 3 bis 4 h nachrühren und engte anschließend das Volumen auf etwa 40 ml ein. Die ausfallenden farblosen Kristalle von 3-Chlor-benzoesäure wurden abgesaugt. Das Filtrat wurde eingeengt und der Rückstand durch Säulenchromatographie (Kieselgel, Toluol/Methanol gereinigt). Man isolierte 3,4 g (53%) 3-(2,3-Dihydro-1-oxido-3-oxo-1,2-benzisothiazol-2-yl)-1-cyano-2-methyl-propen-1, Schmp. 91 bis 93°C.

Analog den Beispielen 1, 2, 3, 14, 17 bzw. 19 lassen sich herstellen:

20. 3-(2,3-Dihydro-1,1-dioxido-3-oxo-6-chlor-1,2-benzisothiazol-2-yl)-1-cyano-2-methyl-propen-1,
21. 3-(2,3-Dihydro-1,1-dioxido-3-oxo-7-chlor-1,2-benzisothiazol-2-yl)-1-cyano-2-methyl-propen-1,
22. 3-(2,3-Dihydro-1,1-dioxido-3-oxo-4-trifluormethyl-1,2-benzisothiazol-2-yl)-1-cyano-2-methyl-propen-1,
23. 3-(2,3-Dihydro-1,1-dioxido-3-oxo-5-trifluormethyl-1,2-benzisothiazol-2-yl)-1-cyano-2-methyl-propen-1,
24. 3-(2,3-Dihydro-1,1-dioxido-3-oxo-4-methyl-1,2-benzisothiazol-2-yl)-1-cyano-2-methyl-propen-1,
25. 3-(2,3-Dihydro-1,1-dioxido-3-oxo-5-propyl-1,2-benzisothiazol-2-yl)-1-cyano-2-methyl-propen-1,
26. 3-(2,3-Dihydro-1,1-dioxido-3-oxo-1,2-benzisothiazol-2-yl)-1-cyano-2-ethyl-propen-1,
27. 3-(2,3-Dihydro-1,1-dioxido-3-oxo-1,2-benzisothiazol-2-yl)-1-cyano-2,3-dimethyl-propen-1,
28. 4-(4-Chlor-phthalimido-2-yl)-1-cyano-2-methyl-buten-1,
29. 4-(5-Chlor-phthalimido-2-yl)-1-cyano-2-methyl-buten-1.

**Patentansprüche für die Vertragsstaaten BE CH DE FR GB IT LI NL SE**

1. Heterocyclisch substituierte Nitrile der Formel I

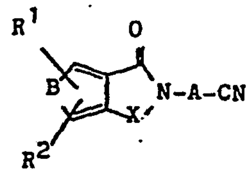

I,

in der
X $SO_2$, CO, S, SO oder $CH_2$,
A einen Alkenylenrest mit bis zu 6 C-Atomen,
B -CH=CH- oder S und
$R^1$ und $R^2$ Wasserstoff, Halogen, $C_{1-3}$-Alkyl, $C_{1-3}$-Alkoxy, Nitro und Trifluormethyl bedeuten.

2. 3-(2,3-Dihydro-1,1-dioxido-3-oxo-1,2-benzisothiazol-2-yl)-1-cyano-2-methyl-propen-1.
3. 3-(2,3-Dihydro-1,1-dioxido-3-oxo-4-chlor-1,2-benzisothiazol-2-yl)-1-cyano-2-methyl-propen-1.
4. 3-(2,3-Dihydro-1,1-dioxido-3-oxo-5-fluor-1,2-benzisothiazol-2-yl)-1-cyano-2-methyl-propen-1.
5. 3-(2,3-Dihydro-1,1-dioxido-3-oxo-5-chlor-1,2-benzisothiazol-2-yl)-1-cyano-2-methyl-propen-1.
6. 4-(2,3-Dihydro-1,1-dioxido-3-oxo-1,2-benzisothiazol-2-yl)-1-cyano-2-methyl-buten-1.
7. Verfahren zur Herstellung der Verbindungen der Formel I gemäß Anspruch 1, *dadurch gekennzeichnet,* daß man

a) eine Verbindung der Formel II

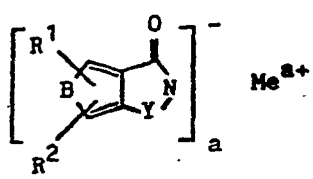

- II,

worin Y dasselbe wie X, ausgenommen SO, ist, Me ein Alkali- oder Erdkalimetall der Wertigkeit a ist und B, $R^1$ und $R^2$ die un Anspruch 1 angegebene Bedeutung besitzen, mit einer Verbindung der Formel III

Hal-A-CN III,

worin A die angegebene Bedeutung hat und Hal ein Halogenatom darstellt oder

b) – falls A einen gegebenenfalls methylverzweigten Alkenylenrest darstellt – eine Verbindung der Formel IV

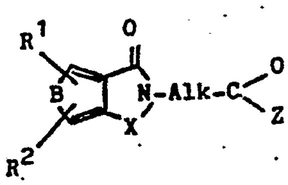

IV,

in der X, B, $R^1$ und $R^2$ die angegebenen Bedeutungen haben, Alk ein Alkylrest mit 1 bis 3 C-Atomen und Z Wasserstoff oder Methyl ist, mit einem Phosphonat der Formel V

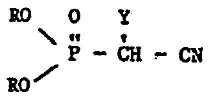

V,

in der R einen Alkylrest mit 1 bis 3 C-Atomen darstellt, und Y Wasserstoff oder Methyl bedeutet, umsetzt und in den so erhaltenen Verbindungen ein gegebenenfalls vorhandenes Schwefelatom – falls gewünscht – oxidiert.

8. Arzneimittel enthaltend eine Verbindung der Formel I gemäß Anspruch 1.

9. Verbindung der Formel I gemäß Anspruch 1 zur Verwendung bei der Bekämpfung von Krankheiten.

**Patentansprüche für den Vertragsstaat AT**

1. Verfahren zur Herstellung heterocyclisch substituierter Nitrile der Formel I

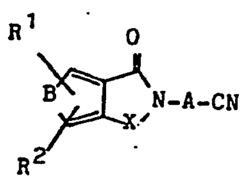

I,

in der
X $SO_2$, CO, S, SO oder $CH_2$,
A einen Alkenylenrest mit bis zu 6 C-Atomen,
B -CH=CH- oder S und
$R^1$ und $R^2$ Wasserstoff, Halogen, $C_{1-3}$-Alkyl, $C_{1-3}$-Alkoxy, Nitro und Trifluormethyl bedeuten,
*dadurch gekennzeichnet,* daß man

a) eine Verbindung der Formel II

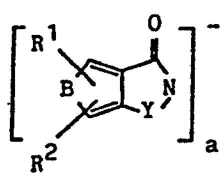

$Me^{a+}$ II,

worin Y dasselbe wie X, ausgenommen SO, ist, Me ein Alkali- oder Erdalkalimetall der Wertigkeit a ist und B, $R^1$ und $R^2$ die angegebene Bedeutung besitzen, mit einer Verbindung der Formel III

Hal-A-CN III,

worin A die angegebene Bedeutung hat und Hal ein Halogenatom darstellt oder

b) – falls A einen gegebenenfalls methylverzweigten Alkenylenrest darstellt – eine Verbindung der Formel IV

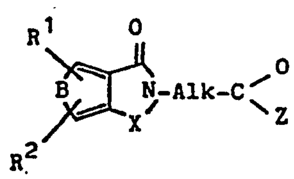

IV,

in der X, B, $R^1$ und $R^2$ die angegebenen Bedeutungen haben, Alk ein Alkylrest mit 1 bis 3 C-Atomen und Z Wasserstoff oder Methyl ist, mit einem Phosphonat der Formel V

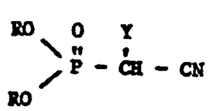

V,

in der R einen Alkylrest mit 1 bis 3 C-Atomen darstellt, und Y Wasserstoff oder Methyl bedeutet, umsetzt

und in den so erhaltenen Verbindungen ein gegebenenfalls vorhandenes Schwefelatom – falls gewünscht – oxidiert.

2. Verfahren gemäß Anspruch 1, *dadurch gekennzeichnet,* daß man 3-(2,3-Dihydro-1,1-dioxido-3-oxo-1,2-benzisothiazol-2-yl)-1-cyano-2-methyl-propen-1 herstellt.

3. Verfahren gemäß Anspruch 1, *dadurch gekennzeichnet,* daß man 3-(2,3-Dihydro-1,1-dioxido-3-oxo-4-chlor-1,2-benzisothiazol-2-yl)-1-cyano-2-methyl-propen-1 herstellt.

4. Verfahren gemäß Anspruch 1, *dadurch gekennzeichnet,* daß man 3-(2,3-Dihydro-1,1-dioxido-3-oxo-5-fluor-1,2-benzisothiazol-2-yl)-1-cyano-2-methyl-propen-1 herstellt.

5. Verfahren gemäß Anspruch 1, *dadurch gekennzeichnet,* daß man 3-(2,3-Dihydro-1,1-dioxido-3-oxo-5-chlor-1,2-benzisothiazol-2-yl)-1-cyano-2-methyl-propen-1 herstellt.

6. Verfahren gemäß Anspruch 1, *dadurch gekennzeichnet,* daß man 4-(2,3-Dihydro-1,1-dioxido-3-oxo-1,2-benzisothiazol-2-yl)-1-cyano-2-methyl-buten-1 herstellt.

**Claims for the contracting States: BE, CH, DE, FR, GB, IT, LI, NL, SE**

1. A heterocyclically substituted nitrile of the formula I

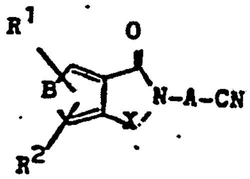

I,

where
X is $SO_2$, CO, S, SO or $CH_2$,
A is an alkenylene radical of up to 6 carbon atoms,
B is -CH=CH- or S, and
$R^1$ and $R^2$ are each hydrogen, halogen, $C_1$-$C_3$-alkyl, $C_1$-$C_3$-alkoxy, nitro or trifluoromethyl.

2. 3-(2,3-Dihydro-1,1-dioxido-3-oxo-1,2-benzisothiazol-2-yl)-1-cyano-2-methylprop-1-ene.
3. 3-(2,3-Dihydro,1,1-dioxido-3-oxo-4-chloro-1,2-benzisothiazol-2-yl)-1-cyano-2-methylprop-1-ene.
4. 3-(2,3-Dihydro-1,1-dioxido-3-oxo-5-fluoro-1,2-benzisothiazol-2-yl)-1-cyano-2-methylprop-1-ene.
5. 3-(2,3-Dihydro-1,1-dioxido-3-oxo-5-chloro-1,2-benzisothiazol-2-yl)-1-cyano-2-methylprop-1-ene.
6. 4-(2,3-Dihydro-1,1-dioxido-3-oxo-1,2-benzisothiazol-2-yl)-1-cyano-2-methylbut-1-ene.
7. A process for the preparation of a compound of the formula I as claimed in claim 1, wherein

a) a compound of the formula II

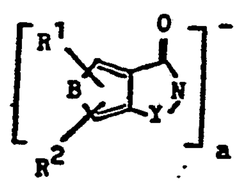

$Me^{a+}$ - II,

where Y has the same meaning as X, with the exception of SO, Me is an alkali metal or alkaline earth metal having a valency a, and B, $R^1$ and $R^2$ have the meanings given in claim 1, is reacted with a compound of the formula III

Hal-A-CN III,

where A has the above meaning and Hal is halogen, or

b) where A is a straight-chain or methyl-branched alkenylene radical, a compound of the formula IV

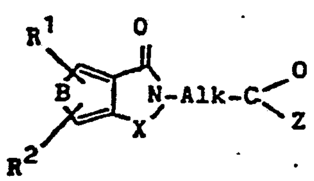

IV,

where X, B, $R^1$ and $R^2$ have the above meanings, Alk is alkyl of 1 to 3 carbon atoms and Z is hydrogen or methyl, is reacted with a phosphonate of the formula V

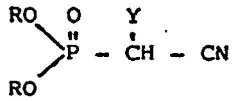 V,

where R is alkyl of 1 to 3 carbon atoms and Y is hydrogen or methyl, and, if desired, any sulfur atom present in the compound thus obtained is oxidized.

8. A pharmaceutical containing a compound of the formula I as claimed in claim 1.

9. A compound of the formula I as claimed in claim 1 for use in tht treatment of disorders.

**Claims for the contracting State: AT**

1. A process for the preparation of a heterocyclically substituted nitrile of the formula I

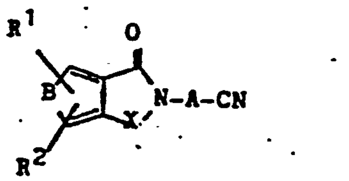 I,

where
X is $SO_2$, CO, S, SO or $CH_2$,
A is an alkenylene radical of up to 6 carbon atoms,
B is -CH=CH- or S, and
$R^1$ and $R^2$ are each hydrogen, halogen, $C_1$-$C_3$-alkyl, $C_1$-$C_3$-alkoxy, nitro or trifluoromethyl, wherein

a) a compound of the formula II

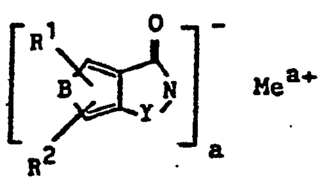 II,

where Y has the same meaning as X, with the exception of SO, Me is an alkali metal or alkaline earth metal having a valency a, and B, $R^1$ and $R^2$ have the above meanings, is reacted with a compound of the formula III,

Hal-A-CN III,

where A has the Above meaning and Hal is halogen, or

b) where A is a straight-chain or methyl-branched alkenylene radical, a compound of the formula IV

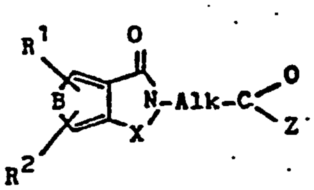 IV,

where X, B, $R^1$ and $R^2$ have the above meanings, Alk is alkyl of 1 to 3 carbon atoms and Z is hydrogen or methyl, is reacted with a phosphonate of the formula V

where R is alkyl of 1 to 3 carbon atoms and Y is hydrogen or methyl, and, if desired, any sulfur atom present in the compound thus obtained is oxidized.

2. A process as claimed in claim 1, wherein 3-(2,3-dihydro-1,1-dioxido-3-oxo-1,2-benzisothiazol-2-yl)-1-cyano-2-methylprop-1-ene is prepared.

3. A process as claimed in claim 1, wherein 3-(2,3-dihydro-1,1-dioxido-3-oxo-4-chloro-1,2-benzisothiazol-2-yl)-1-cyano-2-methylprop-1-ene is prepared.

4. A process as claimed in claim 1, wherein 3-(2,3-dihydro-1,1-dioxido-3-oxo-5-fluoro-1,2-benzisothiazol-2-yl)-1-cyano-2-methylprop-1-ene is prepared.

5. A process as claimed in claim 1, wherein 3-(2,3-dihydro-1,1-dioxido-3-oxo-5-chloro-1,2-benzisothiazol-2-yl)-1-cyano-2-methylprop-1-ene is prepared.

6. A process as claimed in claim 1, wherein 4-(2,3-dihydro-1,1-dioxido-3-oxo-1,2-benzisothiazol-2-yl)-1-cyano-2-methylbut-1-ene is prepared.

**Revendications pour les Etats contractants: BE, CH, DE, FR, GB, IT, LI, NL, SE**

1. Nitriles substitués hétérocycliquement, de formule I

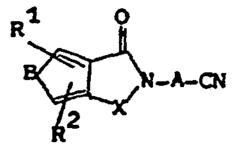 I,

dans laquelle
X représente $SO_2$, CO, S, SO ou $CH_2$,
A représente un reste alcénylène ayant jusqu'à 6 atomes C,
B représente -CH=CH- ou S et
$R^1$ et $R^2$, hydrogène, halogène, alkyle en $C_{1-3}$, alcoxy en $C_{1-3}$, nitro et trifluorométhyle.

2. 3-(2,3-dihydro-1,1-dioxydo-3-oxo-1,2-benzisothiazol-2-yl)-1-cyano-2-méthyl-propène-1.

3. 3-(2,3-dihydro-1,1-dioxydo-3-oxo-4-chloro-1,2-benzisothiazol-2-yl)-1-cyano-2-méthyl-propène-1.

4. 3-(2,3-dihydro-1,1-dioxydo-3-oxo-5-fluoro-1,2-benzisothiazol-2-yl)-1-cyano-2-méthyl-propène-1.

5. 3-(2,3-dihydro-1,1-dioxydo-3-oxo-5-chloro-1,2-benzisothiazol-2-yl)-1-cyano-2-méthyl-propène-1.

6. 4-(2,3-dihydro-1,1-dioxydo-3-oxo-1,2-benzisothiazol-2-yl)-1-cyano-2-méthyl-butène-1.

7. Procédé de préparation des composés de formule I selon la revendication 1, caractérisé par le fait que l'on fait réagir

a) un composé de formule II

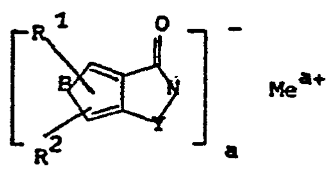 II,

dans laquelle Y est identique à X, excepté SO, Me est un métal alcalin ou alcalinoterreux de valence a et B, $R^1$ et $R^2$ ont la signification donnée dans la revendication 1, avec un composé de formule III

Hal-A-CN III,

où A a la signification donnée plus haut et Hal représente un atome d'halogène

b) – dans le où A représente un reste alcénylène éventuellement ramifié sur méthyle, un composé de formule IV

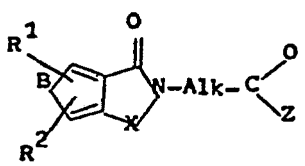 IV,

dans laquelle X, B, $R^1$ et $R^2$ ont les significations susindiquées, Alk est un reste alkyle de 1 à 3 atomes C et Z hydrogène ou méthyle, avec un phosphonate de formule V

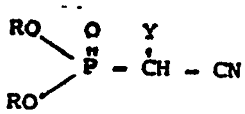 V,

dans laquelle R représente un reste alkyle de 1 à 3 atomes C, et Y hydrogène ou méthyle et, dans les composés ainsi obtenus, on oxyde, si on le souhaite, un atome de soufre éventuellement présent.

8. Médicament contenant un composé de formule I selon la revendication 1.
9. Composé de formule I selon la revendication 1 pour utilisation pour la lutte contre les maladies.

**Revendications pour l'Etat contractant : AT**

1. Procédé de préparation de nitriles substitués hétérocycliquement, de formule I

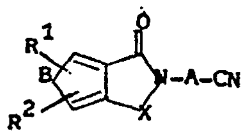

I,

dans laquelle
X représente $SO_2$, CO, S, SO ou $CH_2$,
A représente un reste alcénylène ayant jusqu'à 6 atomes C,
B représente -CH=CH- ou S et
$R^1$ et $R^2$, hydrogène, halogène, alkyle en $C_{1-3}$, alcoxy en $C_{1-3}$, nitro et trifluorométhyle.

caractérisé par le fait que l'on fait réagir

a) un composé de formule II

dans laquelle Y est identique à X, excepté SO, Me est un métal alcalin ou alcalinoterreux de valence a et B, $R^1$ et $R^2$ ont la signification donnée ci-dessus, avec un composé de formule III

Hal-A-CN III,

où A a la signification donnée plus haut et Hal représente un atome d'halogène

b) – dans le cas où A représente un reste alcénylène éventuellement ramifié sur méthyle, un composé de formule IV

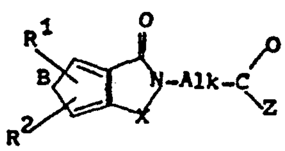

IV,

dans laquelle X, B, $R^1$ et $R^2$ ont les significations susindiquées, Alk est un reste alkyle de 1 à 3 atomes C et Z hydrogène ou méthyle, avec un phosphonate de formule V

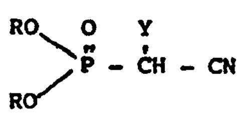

V,

dans laquelle R représente un reste alkyle de 1 à 3 atomes C, et Y hydrogène ou méthyle et, dans les composés ainsi obtenus, on oxyde, si on le souhaite, un atome de soufre éventuellement présent.

2. Procédé selon la revendication 1, caractérisé par le fait que l'on prépare du 3-(2,3-dihydro-1,1-dioxydo-3-oxo-1,2-benzisothiazol-2-yl)-1-cyano-2-méthyl-propène 1.
3. Procédé selon la revendication 1, caractérisé par le fait que l'on prépare du 3-(2,3-dihydro-1,1-dioxydo-3-oxo-4-chloro-1,2-benzisothiazol-2-yl)-1-cyano-2-méthyl-propène 1.
4. Procédé selon la revendication 1, caractérisé par le fait que l'on prépare du 3-(2,3-dihydro-1,1-dioxydo-3-oxo-5-fluoro-1,2-benzisothiazol-2-yl)-1-cyano-2-méthyl-propène-1.

5. Procédé selon la revendication 1, caractérisé par le fait que l'on prépare du 3-(2,3-dihydro-1,1-dioxydo-3-oxo-5-chloro-1,2-benzisothiazol-2-yl)-1-cyano-2-méthyl-propène-1.

6. Procédé selon la revendication 1, caractérisé par le fait que l'on prépare du 4-(2,3-dihydro-1,1-dioxyde-3-oxo-1,2-benzisothiazol-2-yl)-1-cyano-2-méthyl-butène-1.